## Europäisches Patentamt

## European Patent Office

## Office européen des brevets

(11) Veröffentlichungsnummer: **0 355 674**

**A1**

(12) **EUROPÄISCHE PATENTANMELDUNG**

(21) Anmeldenummer: 89115061.7

(22) Anmeldetag: 16.08.89

(51) Int. Cl.⁴: **C07C 305/10 , C11D 1/28**

(30) Priorität: 25.08.88 DE 3828891

(43) Veröffentlichungstag der Anmeldung:
28.02.90 Patentblatt 90/09

(84) Benannte Vertragsstaaten:
ES GR

(71) Anmelder: Henkel Kommanditgesellschaft auf Aktien
Postfach 1100 Henkelstrasse 67
D-4000 Düsseldorf 1(DE)

(72) Erfinder: Behler, Ansgar, Dr.
Siegfriedstrasse 80
D-4250 Bottrop(DE)
Erfinder: Schenker, Gilbert, Dr.
Hermann-Hesse Strasse 5
D-4006 Erkrath-Hochdahl(DE)
Erfinder: Stoll, Gerhard, Dr.
Danziger Strasse 69
D-4052 Korschenbroich(DE)

(54) Vicinal sulfato, oxy-substituierte Fettsäurederivate, Verfahren zu ihrer Herstellung und ihre Verwendung als oberflächenaktive Mittel.

(57) Vicinal sulfato, oxy-substituierte Fettsäurederivate der allgemeinen Formel (I)

$R^1$(vic. $OSO_3M$, $OR^2$)-$COOR^3$    (I)

in der

$R^1$ einen dreibindigen, gesättigten Kohlenwasserstoffrest mit 10 bis 21 Kohlenstoffatomen, der ein Strukturelement der allgemeinen Formel (II)

-$CH(OSO_3M)$-$CH(OR^2)$-    (II)

enthält,

$R^2$ einen Alkyl- bzw. Alkenylrest mit 1 bis 18 Kohlenstoffatomen oder einen Alkylenoxyalkyl- bzw. Alkylenoxyalkenylrest der allgemeinen Formel (III)

-$(C_mH_{2m}O)_n$-$R^2$    (III)

in der m die Zahlen 2 und/oder 3, n eine Zahl von 1 bis 20 und $R^2$ wie oben definiert ist,

M ein Kation aus der Gruppe der Alkalimetallkationen und der gegebenenfalls alkyl- oder hydroxyalkylsubstituierten Ammoniumionen oder ein Äquivalent eines Erdalkalimetallkations und

$R^3$ ein Wasserstoffatom oder eine Alkylgruppe mit 1 bis 12 Kohlenstoffatomen oder ein Kation gemäß der oben für M angegebenen Definition

bedeuten,

lassen sich als gut wirksame oberflächenaktive Mittel einsetzen.

## Vicinal sulfato, oxy-substituierte Fettsäurederivate, Verfahren zu ihrer Herstellung und ihre Verwendung als oberflächenaktive Mittel.

Die Erfindung betrifft vicinal sulfato, oxy-substituierte Fettsäurederivate der allgemeinen Formel (I)

$R^1(vic. OSO_3M, OR^2)\text{-}COOR^3$     (I)

in der

$R^1$ einen dreibindigen, gesättigten Kohlenwasserstoffrest mit 10 bis 21 Kohlenstoffatomen, der ein Strukturelement der allgemeinen Formel (II)

$-CH(OSO_3M)\text{-}CH(OR^2)-$     (II)

enthält,

$R^2$ einen Alkyl- bzw. Alkenylrest mit 1 bis 18 Kohlenstoffatomen oder einen Alkylenoxyalkyl- bzw. Alkylenoxyalkenylrest der allgemeinen Formel (III)

$-(C_mH_{2m}O)_n\text{-}R^2$     (III)

in der m die Zahlen 2 und/oder 3, n eine Zahl von 1 bis 20 und $R^2$ wie oben definiert ist,

M ein Kation aus der Gruppe der Alkalimetallkationen und der gegebenenfalls alkyl- oder hydroxyalkylsubstituierten Ammoniumionen oder ein Aquivalent eines Erdalkalimetallkations

und

$R^3$ ein Wasserstoffatom oder eine Alkylgruppe mit 1 bis 12 Kohlenstoffatomen oder ein Kation gemäß der oben für M angegebenen Definition

bedeuten.

Die technisch wichtigsten anionischen oberflächenaktiven Mittel sind diejenigen, die eine Sulfongruppe als wasserlöslich-machende Gruppe enthalten; bei diesen Tensiden wird zwischen Sulfattensiden und Sulfonattensiden unterschieden.

Sulfattenside sind Halbestersalze der Schwefelsäure. Die wichtigsten Vertreter dieser Untergruppe sind die Alkylethersulfate, die wegen ihrer Glykolethergruppen sehr gut wasserlöslich sind. Typische Beispiele für derartige Sulfattenside sind die aus der US-C 1 985 747 bekannten Alkylglykolethersulfonate, die aus der DE-B 1 075 779 und DE-B 1 081 172 bekannten Alkylglyceryl-ethersulfonate sowie die aus der DE-OS 33 31 513 bekannten Fettalkyl(polyoxyalkyl)-niedrig-alkyl-ethersulfonate.

Es wurde nun gefunden, daß vicinal sulfato, oxy-substituierte Fettsäurederivate der allgemeinen Formel (I) sich in einfacher Weise aus leicht zugänglichen, vicinal hydroxy, oxy-substituierten Fettsäureestern herstellen lassen; dabei ist überraschend, daß bei der Sulfierung der Fettsäureester und der anschließenden Neutralisation der Sulfierungsprodukte die Esterfunktion weitgehend erhalten bleibt, so daß sie, falls unter die Formel (I) fallende vicinal sulfato, oxysubstituierte Fettsäuren bzw. Salze derselben hergestellt werden sollen, mittels geeigneter üblicher Verfahrensmaßnamen verseift werden muß.

Überraschenderweise sind die Verbindungen der Erfindung auch ungebleicht hellfarbig.

Die vicinal sulfato, oxy-substituierten Fettsäurederivate der Erfindung können hergestellt werden, indem man einen vicinal hydroxy, oxy-substituierten Fettsäureester der allgemeinen Formel (IV)

$R^1(vic.OH, OR^2)\text{-}COOR^3$     (IV)

in der $R^1$, $R^2$ bzw. m, n und $R^2$ sowie $R^3$ wie oben definiert sind, in an sich bekannter Weise, z.B. mit Chlorsulfonsäure oder Schwefeltrioxid, sulfatiert und die erhaltenen sulfatierten Verbindungen mit Basen aus der von Alkali- oder Erdalkalihydroxiden, -alkoholaten und -carbonaten sowie von Ammoniak bzw. alkyl- oder hydroxyalkylsubstituierten Aminen gebildeten Gruppe neutralisiert sowie gegebenenfalls die Alkoxycarbonylfunktion $-COOR^3$ verseift.

Die vicinal hydroxy, oxy-substituierten Fettsäureester der allgemeinen Formel (IV) können erhalten werden, in dem man einfach ungesättigte, geradkettige oder verzweigte Carbonsäureester mit den Gruppen $R^1$ und $R^3$ entsprechenden Kohlenstoffzahlen der Acyl- und Alkoxyfunktionen epoxidiert, z.B. gemäß DE-C 30 02 861, und die eingeführte Oxirangruppe mit Alkanolen der Formel $R^2\text{-}OH$ bzw. Anlagerungsprodukten von Ethylenoxid und/oder Propylenoxid an die Alkanole $R^2\text{-}OH$ der Formel $R^2\text{-}(OC_mH_{2m})_n\text{-}OH$ unter Öffnung der Oxirangruppe umsetzt, vgl. gemäß DE-A 33 26 455.

Bevorzugte Ausgangsverbindungen der Formel (IV) sind Fettsäureester, die sich von einfach ungesättigten Fettsäuren mit 11 bis 22 Kohlenstoffatomen natürlicher und/oder synthetischer Herkunft ableiten, z.B. von Undecylen-, Palmitolein, Öl-, Elaidin-, Petroselin-, Gadolein- und Erucasäure. Diese Säuren bzw. Ester werden, wie in der Fettchemie üblich, meist in Form von an den jeweiligen Fettsäuren reichen technischen Gemischen eingesetzt, die ihrerseits z.B. aus pflanzlichen und/oder tierischen Fetten und Ölen, z.B. Rindertalg,Schweineschmalz, Rapsöl, Rüböl, Sojaöl und dergleichen zugänglich sind.

Die bei der Ringöffnung der oben genannten Oxirangruppen eingeführten Reste $R^2$ bzw. $R^2(OC_mH_{2m})_n$-leiten sich ab von gesättigten oder ungesättigten, geradkettigen oder verzweigten Alkoholen mit 1 bis 18 Kohlenstoffatomen bzw. Anlagerungsprodukten von Ethylenoxid, Propylenoxid oder Ethylenoxid und Propylenoxid (bei random- oder block-Verteilung der Propylenoxygruppen) an die vorgenannten Alkohole.

Typische Beispiele für die Alkohole $R^2$-OH sind Methanol, Ethanol, Propanol, Butanol, Pentanol, Hexanol, Heptanol, Octanol, Dodecanol, Tetradecanol, Hexadecanol und Octadecanol einschließlich ihrer Stellungsisomeren und olefinisch ungesättigten Derivate. Besonders bevorzugt sind hier gesättigte oder ungesättigte, geradkettige Alkohole mit 8 bis 18 Kohlenstoffatomen synthetischer und insbesondere natürlicher Herkunft, wie sie aus den vorgenannten natürlichen Rohstoffquellen zugänglich sind, z.B. Capryl-, Caprin-, Lauryl-, Myristyl-, Cetyl-, Stearyl- und Oleylalkohol einschließlich der in der Fettchemie üblicherweise eingesetzten, an den jeweiligen Alkoholen reichen technischen Gemischen derselben.

Die Gruppe $R^3$ in den obigen allgemeinen Formeln ist von Monoalkanolen mit 1 bis 12 Kohlenstoffatomen abgeleitet; typische Vertreter sind den obigen Aufzählungen zu entnehmen.

Typische Vertreter für Kationen bzw. Dikationen der Bedeutung M sind neben Ammonium-, Lithium-, Natrium-, Kalium-, Magnesium- und Calciumionen, Mono-$C_1$-$C_4$-alkyl-, Di-$C_1$-$C_4$-alkyl-, Tri-$C_1$-$C_4$-alkyl-, Monoethanol-, Diethanol- und Triethanolammoniumionen; die Bindung M = $Na^+$ ist besonders bevorzugt.

Gemäß einer bevorzugten Ausführungsform betrifft die Erfindung vicinal sulfato, oxy-substituierte Fettsäurederivate der allgemeinen Formel (I), in der
$R^2$ eine Alkyl- bzw. Alkenylrest mit 8 bis 18 Kohlenstoffatomen,
$R^3$ eine Alkylgruppe mit 1 bis 4 Kohlenstoffatomen,
m die Zahl 2 und
n eine Zahl von 2 bis 10
bedeuten, sowie $R^1$ wie oben definiert, vorzugsweise ein dreibindiger gesättigter Kohlenwasserstoffrest mit 17 Kohlenstoffatomen ist, sowie Verfahren zu ihrer Herstellung. Typische Beispiele für bevorzugte Bedeutungen der Gruppen $R^1$, $R^2$ und $R^3$ ergeben sich aus den obigen Erläuterungen.

Die Sulfatierung der Fettsäureester der allgemeinen Formel (IV) erfolgt mit den üblichen Sulfatierungsmitteln wie Chlorsulfonsäure und - bevorzugt- mit gasförmigem Schwefeltrioxid bei Temperaturen von 20 bis 100, insbesondere von 5 bis 40 °C. Diese Umsetzung läßt sich in üblichen, für die Sulfatierung bzw. Sulfonierung von Fettalkoholen, Fettsäureestern, Alkylbenzol oder Olefinen geeigneten Reaktoren kontinuierlich oder diskontinuierlich durchführen, wobei für die kontinuierliche Sulfatierung Reaktoren vom Typ der Fallfilmreaktoren besonders geeignet sind. Dabei wird das Schwefeltrioxid mit Luft oder Stickstoff verdünnt und vorzugsweise in Form eines 1 bis 10 Vol-% Schwefeltrioxid enthaltenden Gasgemisches mit den Carbonsäureestern der Formel (IV) in Berührung gebracht. Anschließend wird das rohe Sulfatierungsprodukt in eine wäßrige Lösung der oben genannten Basen eingeleitet, wobei die Base in einer Menge von 1 bis 1,2 Mol pro Mol angelagertem Schwefeltrioxid zur Neutralisation des im Sulfatierungsprodukt gelöstem gasförmigen Schwefeltrioxids vorliegen sollte. Bevorzugte beträgt das Einsatzmolverhältnis von zu sulfatierendem Carbonsäureester zu Schwefeltrioxid 1:1 bis 1:1,3.

Die so erhaltenen vicinal sulfato, oxy-substituierten Fettsäureester können, falls erwünscht, anschließend zu den entsprechenden Fettsäuren verseift werden. Frei Fettsäuren der allgemeinen Formel I können in übrigen auch erhalten werden in dem man zunächst die Fettsäureester der allgemeinen Formel (IV) verseift und anschließend sulfatiert.

Die Struktur der vicinal sulfato, oxy-substituierten Fettsäurederivate der allgemeinen Formel (I) ist noch nicht endgültig geklärt. Vorliegende Analyseergebnisse deuten auf eine sekundäre Sulfatgruppe hin, wobei alpha-Estersulfonierungen, wenn überhaupt, nur untergeordnet stattgefunden haben. Herstellungsbedingt läßt sich im übrigen auch nicht angeben, an welchen Kohlenstoffatomen des ursprünglichen Oxiranringes sich die Sulfato- bzw. Oxysubstituenten befinden.

Schließlich betrifft die Erfindung die Verwendung von vicinal sulfato, oxy-substituierten Fettsäurederivaten der allgemeinen Formel (I) als oberflächenaktive Mittel.

Die Erfindung wird im folgenden anhand von bevorzugten Ausführungsbeispielen näher erläutert.

Beispiel 1.


Sulfierung von 9(10)-Laurylalkoholoctaglykol-oxy-10(9)-hydroxystearinsäuremethylester.


Als Ausgangsprodukt diente das Umsetzungsprodukt eines handelsüblichen Epoxids eines technischen Ölsäuremethylesters mit dem Anlagerungsprodukt von 8 Mol Ethylenoxid an einen ebenfalls handelsüblichen Laurylalkohol.

Der eingesetzte Ölsäuremethylester wies eine Verseifungszahl von 192 bis 197 und eine Jodzahl von 84 bis 92 sowie die folgende Kettenverteilung (in %) auf:

$C_{10}$ 0-0,5

$C_{12}$ 0-2

$C_{14}$ 0-5

$C_{15}$ 0-1

$C_{16}$ 4-6 gesättigt

4 bis 6 ungesättigt

$C_{17}$ 1-3

$C_{18}$ 1-3 gesättigt

63 bis 73 einfach ungesättigt

7 bis 12 zweifach ungesättigt

0 bis 2 dreifach ungesättigt

$C_{20}$ 0-2

$C_{22}$ 0-2

Der Epoxid-Sauerstoffgehalt des daraus hergestellten Epoxids betrug 4,61 %.

Der eingesetzte Octaglykollaurylalkohol wies die folgende Kettenverteilung(in %) auf:

$C_{10}$ 0-2

$C_{12}$ ≧ 97

$C_{14}$ 0-3.

150 g(0,21 Mol) 9(10)Laurylalkoholoctaglykol-oxy-10(9)-hydroxystearinsäuremethylester, erhalten in der oben beschriebenen Weise, wurden in einem Labor-Standreaktor erwärmt. Innerhalb von 50 min wurden 18,3 g Schwefeltrioxid (0,23 Mol, erzeugt durch Verdampfen von 65%-igem Oleum), mit Stickstoff verdünnt auf einen Gehalt von 5 Vol.-% Schwefeltrioxid im Stickstoffstrom, eingeleitet. Nach Beendigung der Reaktion wurde das saure Sulfierprodukt mit wäßriger, 25%-iger Natriumhydroxid-Lösung neutralisiert (8,7 g = 0,22 Mol Natriumhydroxid). Das erhaltene Reaktionsprodukt war in Wasser zu einer ca. 35%-igen Lösung löslich.

Beispiele 2 bis 5.

Analog zu der in Beispiel 1 beschriebenen Weise wurden die in Tabelle 1 zusammengefaßten und über die Bedeutungen von $R^1$, $R^2$, $R^3$, m, n und M gekennzeichneten vicinal sulfato, oxysubstituierten Fettsäurederivate hergestellt.

Die analytischen Kenndaten der Verbindungen in den Beispielen 1 bis 5 sind in Tabelle 2 zusammengefaßt.

Tabelle 1

| Hergestellte Verbindungen der Formel (I) | | | | | | |
|---|---|---|---|---|---|---|
| $R^1$(vic.$OSO_3$M, $OR^2$)-$COOR^3$ | | | | | | |
| ($R^2$ = -$(C_mH_{2m}O)_n$-$R^2$) | | | | | | |
| Beispiel | $R^1$ | $R^2$ | $R^3$ | m | n | M |
| 1 | $C_{18}$ | $C_{12}$ | $C_1$ | 2 | 8 | Na |
| 2 | $C_{18}$ | $C_{10}$ | $C_1$ | 2 | 2,9 | Na |
| 3 | $C_{18}$ | $C_{12}$ | $C_1$ | 2 | 6 | Na |
| 4 | $C_{18}$ | $C_8$ | $C_1$ | 2 | 4 | Na |
| 5 | $C_{18}$ | $C_{18/18}$[1] | $C_1$ | 2 | 5 | Na |

1) Techn. Gemisch von gesättigten und einfach ungesättigten $C_{18}$-Fettalkylverbindungen.

Tabelle 2

| Analytische Daten der hergestellten Verbindungen der Formel I | | | |
|---|---|---|---|
| Beispiel | WAS[1] (%) | US[2] (%) | SG[3] (%) |
| 1 | 23,2 | 8,2 | 71,2 |
| 2 | 27 | 6,6 | 71,2 |
| 3 | 27 | 6,6 | 78,1 |
| 4 | 26,7 | 10,0 | 69,4 |
| 5 | 20 | 12,0 | 60,1 |

1) Waschaktive Substanz nach Epton
2) Unsulfierte Anteile
3) Sulfierungsgrad

## Ansprüche

1. Vicinal sulfato, oxy-substituierte Fettsäurederivate der allgemeinen Formel (I)

$R^1$(vic. $OSO_3M$, $OR^2$)-$COOR^3$    (I)

in der

$R^1$ einen dreibindigen, gesättigten Kohlenwasserstoffrest mit 10 bis 21 Kohlenstoffatomen, der ein Strukturelement der allgemeinen Formel (II)

-$CH(OSO_3M)$-$CH(OR^2)$-    (II)

enthält,

$R^2$ einen Alkyl- bzw. Alkenylrest mit 1 bis 18 Kohlenstoffatomen oder einen Alkylenoxyalkyl- bzw. Alkylenoxyalkenylrest der allgemeinen Formel (III)

-$(C_mH_{2m}O)_n$-$R^2$    (III)

in der m die Zahlen 2 und/oder 3, n eine Zahl von 1 bis 20 und $R^2$ wie oben definiert ist,

M ein Kation aus der Gruppe der Alkalimetallkationen und der gegebenenfalls alkyl- oder hydroxyalkylsubstituierten Ammoniumionen oder ein Äquivalent eines Erdalkalimetallkations

und

$R^3$ ein Wasserstoffatom oder eine Alkylgruppe mit 1 bis 12 Kohlenstoffatomen oder ein Kation gemäß der oben für M angegebenen Definition

bedeuten.

2. Vicinal sulfato, oxy-substituierte Fettsäurederivate der allgemeinen Formel (I) nach Anspruch 1, in der

$R^2$ eine Alkyl- bzw. Alkenylrest mit 8 bis 18 Kohlenstoffatomen,

$R^3$ eine Alkylgruppe mit 1 bis 4 Kohlenstoffatomen,

m die Zahl 2 und

n eine Zahl von 2 bis 10

bedeuten, sowie $R^1$ wie oben definiert ist.

3. Vicinal sulfato, oxy-substituierte Fettsäurederivate der allgemeinen Formel (I) nach Anspruch 1 oder 2, in der

$R^1$ ein dreibindiger gesättigter Kohlenwasserstoffrest mit 18 Kohlenstoffatomen bedeutet.

4. Verfahren zur Herstellung von vicinal sulfato, oxysubstituierten Fettsäurederivaten der allgemeinen Formel (I)

$R^1$(vic. $OSO_3M$, $OR^2$)-$COOR^3$    (I)

in der

$R^1$ einen dreibindigen, gesättigten Kohlenwasserstoffrest mit 10 bis 21 Kohlenstoffatomen, der ein Strukturelement der allgemeinen Formel (II)

-$CH(OSO_3M)$-$CH(OR^2)$-    (II)

enthält,

$R^2$ einen Alkyl- bzw. Alkenylrest mit 1 bis 18 Kohlenstoffatomen oder einen Alkylenoxyalkyl- bzw. Alkylenoxyalkenylrest der allgemeinen Formel (III)

$-(C_mH_{2m}O)_n-R^2$    (III)

in der m die Zahlen 2 und/oder 3, n eine Zahl von 1 bis 20 und $R^2$ wie oben definiert ist,

M ein Kation aus der Gruppe der Alkalimetallkationen und der gegebenenfalls alkyl- oder hydroxyalkylsubstituierten Ammoniumionen oder ein Äquivalent eines Erdalkalimetallkations

und

$R^3$ ein Wasserstoffatom oder eine Alkylgruppe mit 1 bis 12 Kohlenstoffatomen oder ein Kation gemäß der oben für M angegebenen Definition

bedeuten,

dadurch gekennzeichnet, daß man vicinal hydroxy, oxy-substituierte Fettsäureester der allgemeinen Formel (IV)

$R^1(vic.OH, OR^2)-COOR^3$    (IV)

in der $R^1$, $R^2$ bzw. m, n und $R^2$ sowie $R^3$ wie oben definiert sind, in an sich bekannter Weise sulfatiert und die erhaltenen sulfatierten Verbindungen mit Basen der von Alkali- oder Erdalkalihydroxiden, -alkoholaten und -carbonaten sowie von Ammoniak bzw. alkyl- oder hydroxyalkylsubstituierten Aminen gebildeten Gruppe neutralisiert sowie gegebenenfalls die Alkoxycarbonylfunktion -COOR³ verseift.

5. Verfahren nach Anspruch 4, dadurch gekennzeichnet, daß man vicinal hydroxy, oxy-substituierte Fettsäureester der allgemeinen Formel (IV) verwendet, in der

R' bzw. $R^1(vic.OH, OR^2)$ wie oben definiert ist und

$R^2$ einen Alkyl- bzw. Alkenylrest mit 8 bis 18 Kohlenstoffatomen,

$R^3$ eine Alkylgruppe mit 1 bis 4 Kohlenstoffatomen,

m die Zahl 2 und

n eine Zahl von 2 bis 10

bedeuten.

6. Verfahren nach Anspruch 4 oder 5, dadurch gekennzeichnet, daß man einen vicinal hydroxy, oxy-substituierten Fettsäureester der allgemeinen Formel (IV) verwendet, in der

$R^1$ einen dreibindigen, gesättigten Kohlenwasserstoffrest mit 17 Kohlenstoffatomen bedeutet, wobei $R^2$, $R^3$, m und n wie oben definiert sind.

7. Verfahren nach mindestens einem der Ansprüche 4 bis 6, dadurch gekennzeichnet, daß man mit Schwefeltrioxid bei molaren Einsatzverhältnissen von Fettsäureestern der allgemeinen Formel (II) zu Schwefeltrioxid von 1:1 bis 1:1,3 sulfatiert.

8. Verwendung der vicinal sulfato, oxy-substituierten Fettsäurederivate der allgemeinen Formel (I) nach mindestens einem der Ansprüch 1 bis 3 als oberflächenaktive Mittel.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | BERICHTE VOM INTERNATIONAL KONGRESS FUER GRENZFLAECHENAKTIVE STOFFE International Congress on Surface Adive Substances Hansez, Band 5, 9.-13. September 1968, Seiten 63-70, München, D; K.T. ACHAYA: "Structure vs. surfactance in alkyl and aralkyl compounds" * das ganze Dokument *  --- | 1-4,6,8 | C 07 C 305/10 C 11 D   1/28 |
| A | US-A-2 032 313  (H. BERTSCH et al.) * das ganze Dokument *  --- | 1-8 | |
| A | US-A-2 007 492  (H. BERTSCH et al.) * Seiten 1,2 *  --- | 1,8 | |
| A | US-A-1 823 815  (H. BERTSCH et al.) * Ansprüche *  --- | 1 | |
| A | US-A-1 796 801  (F. MUENZ) * Seiten 1,2 *  ----- | 1,8 | RECHERCHIERTE SACHGEBIETE (Int. Cl.5)  C 07 C 305/00 C 11 D   1/00 |

Der vorliegende Recherchenbericht wurde für alle Patentansprüche erstellt

| Recherchenort | Abschlußdatum der Recherche | Prüfer |
|---|---|---|
| BERLIN | 18-10-1989 | RUFET J.M.A. |

EPO FORM 1503 03.82 (P0403)